(19) Europäisches Patentamt
European Patent Office
Office européen des brevets

(11) **EP 4 218 583 A1**

(12) **EUROPEAN PATENT APPLICATION**
published in accordance with Art. 153(4) EPC

(43) Date of publication:
**02.08.2023 Bulletin 2023/31**

(21) Application number: **21871977.1**

(22) Date of filing: **30.07.2021**

(51) International Patent Classification (IPC):
***A61B 6/00*** (2006.01)

(52) Cooperative Patent Classification (CPC):
**A61B 6/00**

(86) International application number:
**PCT/JP2021/028493**

(87) International publication number:
**WO 2022/064845 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.09.2020 JP 2020161415**

(71) Applicant: **FUJIFILM Corporation
Tokyo 106-8620 (JP)**

(72) Inventor: **TSUJI, Tetsuya
Ashigarakami-gun, Kanagawa 258-8538 (JP)**

(74) Representative: **Hughes, Andrea Michelle
Dehns Germany
Theresienstraße 6-8
80333 München (DE)**

(54) **SETTING DEVICE, SETTING METHOD, AND SETTING PROGRAM**

(57)     A CPU of a setting device acquires imaging part information indicating an imaging part of a subject whose radiographic image is captured by radiation emitted from a radiation emitting device. In addition, in a case in which body thickness information indicating a body thickness of the subject in a direction in which the radiation is transmitted is acquired from a detector that comes into contact with the subject and detects the body thickness, the CPU derives imaging conditions corresponding to the body thickness indicated by the body thickness information and the imaging part indicated by the imaging part information and sets the imaging conditions in the radiation emitting device.

## FIG. 6

EP 4 218 583 A1

**Description**

BACKGROUND OF THE INVENTION

1. Field of the Invention

The present disclosure relates to a setting device, a setting method, and a setting program.

2. Description of the Related Art

In general, in a case in which a radiographic image of a subject is captured by radiation emitted from a radiation emitting device, imaging conditions for emitting the radiation are set for the radiation emitting device. For example, JP2006-218142A discloses a technique that sets imaging conditions corresponding to an imaging part and a body thickness of a subject.

SUMMARY OF THE INVENTION

However, as a method for detecting the body thickness of the subject, a detection method which uses a detector using ultrasonic waves or a laser or a TOF camera is known. In these detection methods, for example, in a case in which the clothes of the subject are loose, a gap occurs between the clothes and a body surface of the subject. In some cases, it is not possible to detect the body surface hidden in the gap. Further, in some cases, it is not possible to set the part to be detected in the subject in detail in the detector using ultrasonic waves or a laser. In addition, it is difficult to specify a region of interest for specifying a detection position according to the part in the TOF camera. Therefore, in some cases, it is difficult to specify the detection position. Furthermore, for example, in the TOF camera, in some cases, a large amount of noise occurs, which causes a problem in reproducibility.
As described above, in some cases, it is difficult to accurately measure the body thickness of the imaging part of the subject. In some cases, since it is not possible to accurately detect the body thickness, it is not possible to set appropriate imaging conditions in the radiation emitting device. Therefore, in the technique disclosed in JP2006-218142A, in some cases, it is difficult to set the imaging conditions.
The present disclosure has been made in view of the above circumstances and provides a setting device, a setting method, and a setting program that can easily set imaging conditions corresponding to an imaging part and a body thickness of a subject in a radiation emitting device.
According to a first aspect of the present disclosure, there is provided a setting device comprising at least one processor. The processor acquires imaging part information indicating an imaging part of a subject whose radiographic image is captured by radiation emitted from a radiation emitting device. In a case in which body thickness information indicating a body thickness of the subject in a direction in which the radiation is transmitted is acquired from a detector that comes into contact with the subject and detects the body thickness, the processor derives imaging conditions corresponding to the body thickness indicated by the body thickness information and the imaging part indicated by the imaging part information and sets the imaging conditions in the radiation emitting device.
According to a second aspect of the present disclosure, in the setting device according to the first aspect, the detector may be integrated with an imaging table for capturing the radiographic image.
According to a third aspect of the present disclosure, in the setting device according to the first aspect or the second aspect, the processor may wirelessly acquire the body thickness information from the detector.
According to a fourth aspect of the present disclosure, in the setting device according to any one of the first to third aspects, the detector may be a digital caliper.
According to a fifth aspect of the present disclosure, in the setting device according to any one of the first to third aspects, the detector may be a digital measure.
According to a sixth aspect of the present disclosure, in the setting device according to any one of the first to fifth aspects, the detector may include a reference measurement device that is provided at a reference position and a pair of measurement devices that comes into contact with both sides of a portion in which the body thickness of the subject is measured, and the processor may acquire positional information indicating a position of each of the pair of measurement devices as the body thickness information and derives the body thickness on the basis of the body thickness information and the reference position.
According to a seventh aspect of the present disclosure, in the setting device according to any one of the first to sixth aspects, the imaging conditions may be at least one of a tube voltage value, a tube current value, an irradiation time, or a mAs value.
According to an eighth aspect of the present disclosure, in the setting device according to any one of the first to seventh aspects, the processor may acquire the imaging part information from an imaging menu.

In addition, according to a ninth aspect of the present disclosure, there is provided a setting processing method executed by a processor. The setting processing method comprises: acquiring imaging part information indicating an imaging part of a subject whose radiographic image is captured by radiation emitted from a radiation emitting device; and deriving, in a case in which body thickness information indicating a body thickness of the subject in a direction in which the radiation is transmitted is acquired from a detector that comes into contact with the subject and detects the body thickness, imaging conditions corresponding to the body thickness indicated by the body thickness information and the imaging part indicated by the imaging part information and setting the imaging conditions in the radiation emitting device.

Further, according to a tenth aspect of the present disclosure, there is provided a setting program that causes a processor to execute a process comprising: acquiring imaging part information indicating an imaging part of a subject whose radiographic image is captured by radiation emitted from a radiation emitting device; and deriving, in a case in which body thickness information indicating a body thickness of the subject in a direction in which the radiation is transmitted is acquired from a detector that comes into contact with the subject and detects the body thickness, imaging conditions corresponding to the body thickness indicated by the body thickness information and the imaging part indicated by the imaging part information and setting the imaging conditions in the radiation emitting device.

According to the present disclosure, it is possible to easily set the imaging conditions corresponding to the imaging part and body thickness of the subject.

BRIEF DESCRIPTION OF THE DRAWINGS

Fig. 1 is a diagram schematically illustrating an example of an overall configuration of a radiography system according to an embodiment.
Fig. 2 is a diagram illustrating an example of a detector and a body thickness detection method according to the embodiment.
Fig. 3 is a block diagram illustrating an example of a configuration of a console according to the embodiment.
Fig. 4 is a functional block diagram illustrating an example of a functional configuration of the console according to the embodiment.
Fig. 5 is a diagram illustrating an example of correspondence relationship information indicating a correspondence relationship among a body thickness, a tube voltage value, and a mAs value.
Fig. 6 is a flowchart illustrating an example of a flow of a setting process of the console according to the embodiment.
Fig. 7A is a diagram illustrating an example of a detector as a foldable digital caliper which is provided integrally with an imaging table.
Fig. 7B is a diagram illustrating an example of the detector as the foldable digital caliper which provided integrally with the imaging table.
Fig. 7C is a diagram illustrating an example of the detector as the foldable digital caliper which is provided integrally with the imaging table.
Fig. 7D is a diagram illustrating an example of the detector as the foldable digital caliper which is provided integrally with the imaging table.
Fig. 8A is a diagram illustrating an example of the detector as a digital measure which is provided integrally with the imaging table.
Fig. 8B is a diagram illustrating an example of the detector as the digital measure which is provided integrally with the imaging table.
Fig. 8C is a diagram illustrating an example of the detector as the digital measure which is provided integrally with the imaging table.
Fig. 9 is a diagram illustrating an example of a detector comprising a reference measurement device and a pair of measurement devices.

DESCRIPTION OF THE PREFERRED EMBODIMENTS

Hereinafter, embodiments of the invention will be described in detail with reference to the drawings. In addition, each of the embodiments does not limit the invention.

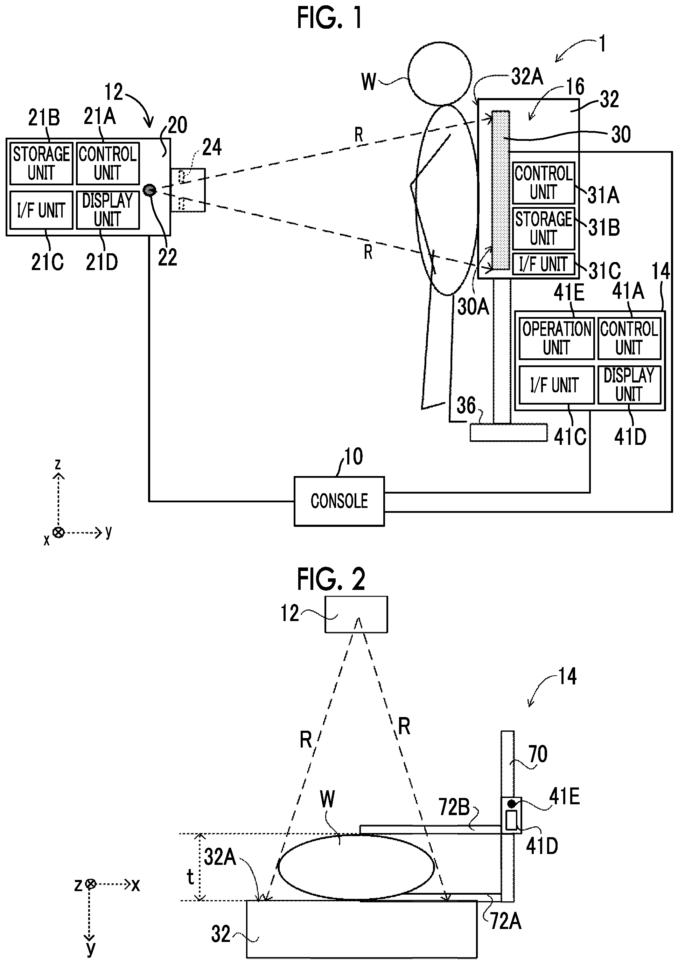

First, an example of an overall configuration of a radiography system according to this embodiment will be described. Fig. 1 is a diagram illustrating an example of the overall configuration of a radiography system 1 according to this embodiment. As illustrated in Fig. 1, the radiography system 1 according to this embodiment comprises a console 10, a radiation emitting device 12, a detector 14, and a radiography apparatus 16. The console 10 according to this embodiment is an example of a setting device according to the present disclosure. In addition, Fig. 1 illustrates an aspect in which a radiographic image is captured in a state in which a subject W is standing up (standing state). However, the state of the subject W is not limited. For example, the subject W may be in a state (sitting state) in which it is sitting on a chair including a wheelchair or in a state in which it lies on an imaging table 32 (lying state).

The radiation emitting device 12 according to this embodiment comprises a radiation source 20 that irradiates the subject W, which is an example of an object to be imaged, with radiation R, such as X-rays, and a collimator 24 that limits an irradiation field of the radiation R emitted from the radiation source 20. In addition, the radiation emitting device 12 comprises a control unit 21A, a storage unit 21B, an interface (I/F) unit 21C, and a display unit 21D.

The control unit 21A controls the radiation source 20 and the collimator 24 under the control of the console 10. The control unit 21A comprises a central processing unit (CPU), a read only memory (ROM), and a random access memory (RAM) which are not illustrated. Various programs, which include an irradiation processing program for causing the radiation source 20 to irradiate the subject W with the radiation R in the capture of a radiographic image and are executed by the CPU, are stored in the ROM in advance. The RAM temporarily stores various types of data.

For example, various types of information are stored in the storage unit 21B. Specific examples of the storage unit 21B include a hard disk drive (HDD) and a solid state drive (SSD). The I/F unit 21C transmits and receives various types of information to and from the console 10 using wireless communication or wired communication. The irradiation emitting device 12 receives imaging conditions (which will be described in detail below) derived by the console 10 through the I/F unit 21C. The display unit 21D is used to display imaging conditions such as a tube voltage and a mAs value set by the console 10. A liquid crystal display (LCD) is given an example of the display unit 21D.

A method by which a user, such as a doctor or a technician, instructs the radiation emitting device 12 to emit the radiation R is not limited. For example, in a case in which the radiation emitting device 12 comprises an irradiation button or the like, the user, such as a radiology technician, may input an instruction to emit the radiation R with the irradiation button such that the radiation R is emitted from the radiation emitting device 12. Further, for example, the user, such as the radiology technician, may operate the console 10 to input the instruction to emit the radiation R such that the radiation R is emitted from the radiation emitting device 12.

In the radiation emitting device 12, in a case in which an instruction to emit the radiation R is received, the control unit 21A directs the radiation source 20 to emit the radiation R from a focus 22 of a radiation tube according to the imaging conditions set by the console 10. For example, in this embodiment, an irradiation field has a rectangular shape. Therefore, a rectangular-pyramid-shaped region that has the focus 22 as the apex and the irradiation field as the base is irradiated with the radiation R emitted from the focus 22.

The radiography apparatus 16 comprises a radiation detector 30, a control unit 31A, a storage unit 31B, and an I/F unit 31C.

The radiation detector 30 has a function of generating a radiographic image. As illustrated in Fig. 1, the radiation detector 30 is disposed in the imaging table 32. In the radiography apparatus 16 according to this embodiment, in a case in which imaging is performed, the subject W is positioned on an imaging surface 32A of the imaging table 32 by the user.

The radiation detector 30 detects the radiation R transmitted through the subject W and the imaging table 32, generates a radiographic image on the basis of the detected radiation R, and outputs image data indicating the generated radiographic image. The type of the radiation detector 30 according to this embodiment is not particularly limited. For example, the radiation detector 30 may be an indirect-conversion-type radiation detector that converts the radiation R into light and converts the converted light into charge or a direct-conversion-type radiation detector that directly converts the radiation R into charge.

The control unit 31A controls the overall operation of the radiography apparatus 16 under the control of the console 10. The control unit 31A comprises a CPU, a ROM, and a RAM which are not illustrated. For example, various programs, which include an imaging processing program for performing control related to the capture of radiographic images and are executed by the CPU, are stored in the ROM in advance. The RAM temporarily stores various types of data.

For example, the image data of the radiographic image captured by the radiation detector 30 and various other types of information are stored in the storage unit 31B. An HDD or an SSD is given as a specific example of the storage unit 31B. The I/F unit 31C transmits and receives various types of information to and from the console 10 using wireless communication or wired communication. The image data of the radiographic image captured by the radiation detector 30 is transmitted to the console 10 through the I/F unit 31C by wireless communication or wired communication.

In addition, the detector 14 has a function of coming into contact with the subject W and detecting a body thickness of the subject W in a direction in which the radiation R is transmitted. As illustrated in Fig. 1, the detector 14 according to this embodiment comprises a control unit 41A, an I/F unit 41C, a display unit 41D, and an operation unit 41E.

The control unit 41A has a function of controlling the operation of the detector 14 in the measurement of the body thickness of the subject W by a technician. The control unit 41A comprises a CPU, a ROM, and a RAM which are not illustrated. For example, various programs including a detection processing program executed by the CPU are stored in the ROM in advance. The RAM temporarily stores various types of data including a detection value of the body thickness of the subject W.

The I/F unit 41C transmits and receives various types of information including the detection result of the body thickness of the subject W to and from the console 10 using wireless communication or wired communication. The display unit 41D is used to display, for example, the detection result of the body thickness of the subject W. A liquid crystal display (LCD) is given an example of the display unit 21D.

For example, the detector 14 according to this embodiment is a digital caliper and comprises a main scale 70, a jaw 72A, and a jaw 72B as illustrated in Fig. 2. The jaw 72A is provided at one end of the main scale 70. Meanwhile, the display unit 41D and the operation unit 41E are provided in the jaw 72B and are moved along the main scale 70. An interval between the jaw 72A and the jaw 72B is displayed on the display unit 41D. In a case in which the operation unit 41E is operated, information indicating the interval between the jaw 72A and the jaw 72B in a case in which the operation unit 41E is operated is output to the console 10. For example, a material, such as a resin, which has a linear expansion coefficient larger than that of metal and is lightweight, can be used as a material forming the detector 14 as the digital caliper. The use of this material makes it possible to reduce the weight of the detector 14 even in a case in which the main scale 70, the jaw 72A, and the jaw 72B are relatively large.

Meanwhile, the console 10 according to this embodiment has a function of controlling the radiation emitting device 12 and the radiography apparatus 16 using, for example, an imaging order and various types of information acquired from a radiology information system (RIS) (not illustrated) or the like through a wireless communication local area network (LAN) or the like.

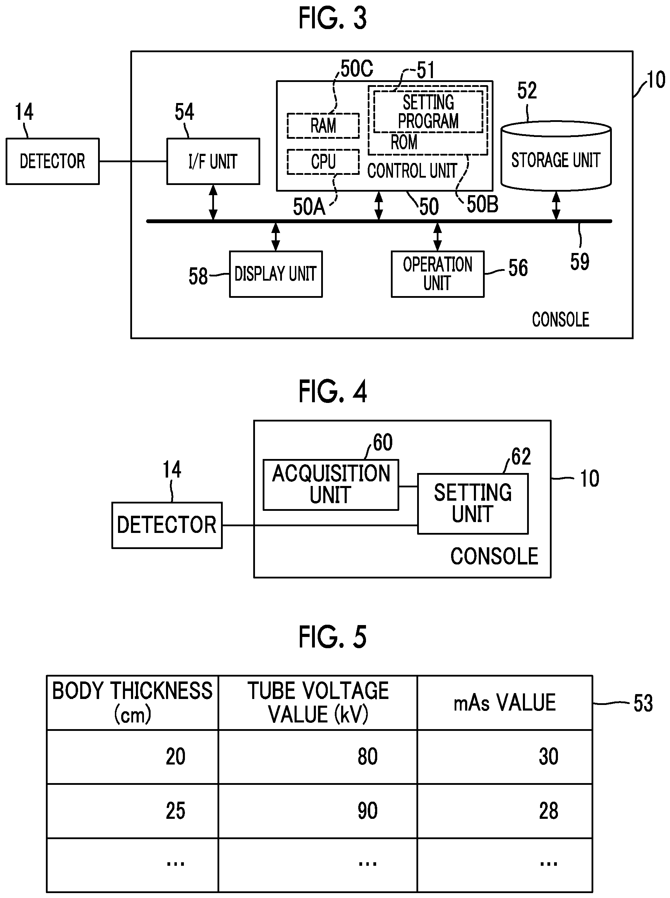

For example, the console 10 according to this embodiment is a server computer. As illustrated in Fig. 3, the console 10 comprises a control unit 50, a storage unit 52, an I/F unit 54, an operation unit 56, and a display unit 58. The control unit 50, the storage unit 52, the I/F unit 54, the operation unit 56, and the display unit 58 are connected to each other through a bus 59, such as a system bus or a control bus, such that they can transmit and receive various types of information.

The control unit 50 according to this embodiment controls the overall operation of the console 10. The control unit 50 comprises a CPU 50A, a ROM 50B, and a RAM 50C. For example, various programs including a setting program 51 executed by the CPU 50A are stored in the ROM 50B in advance. The RAM 50C temporarily stores various types of data. The CPU 50A according to this embodiment is an example of a processor according to the present disclosure. In addition, the setting program 51 according to this embodiment is an example of a setting program according to the present disclosure.

For example, the image data of the radiographic image captured by the radiography apparatus 16 and various types of information including the imaging order acquired from the RIS are stored in the storage unit 52. An HDD or an SSD is given as a specific example of the storage unit 52.

The operation unit 56 is used by the user to designate an imaging menu corresponding to an imaging order and to input instructions related to the capture of a radiographic image including an instruction to emit the radiation R, various types of information, and the like. The operation unit 56 is not particularly limited. Examples of the operation unit 56 include various switches, a touch panel, a touch pen, and a mouse. The display unit 58 is used to display various types of information. In addition, the operation unit 56 and the display unit 58 may be integrated into a touch panel display.

The console 10 displays a plurality of types of imaging menus prepared in advance on the display unit 58 such that one of the menus can be selected. The user selects one imaging menu that is matched with the content of the imaging order through the operation unit 56. In this embodiment, the imaging menu is predetermined for each of imaging parts, such as the head, the chest, the abdomen, and the spine, and the user selects an imaging part to select an imaging menu. Therefore, the console 10 receives the designation of the imaging menu.

The I/F unit 54 transmits and receives various types of information to and from the radiation emitting device 12, the radiography apparatus 16, and the RIS (not illustrated) using wireless communication or wired communication. In the radiography system 1 according to this embodiment, the console 10 receives the image data of the radiographic image captured by the radiography apparatus 16 from the radiography apparatus 16 through the I/F unit 54, using wireless communication or wired communication.

In addition, Fig. 4 is a functional block diagram illustrating an example of a functional configuration of the console 10 according to this embodiment. As illustrated in Fig. 4, the console 10 comprises an acquisition unit 60 and a setting unit 62. For example, in the console 10 according to this embodiment, the CPU 50A of the control unit 50 executes the setting program 51 stored in the ROM 50B to function as the acquisition unit 60 and the setting unit 62.

The acquisition unit 60 has a function of acquiring imaging part information indicating an imaging part of the subject W. For example, in this embodiment, the imaging part information is acquired from the received imaging menu. In addition, the method by which the acquisition unit 60 acquires the imaging part information is not particularly limited. For example, in a case in which the imaging part information is included in the imaging order, the acquisition unit 60 may acquire the imaging part information from the imaging order. The imaging part information acquired by the acquisition unit 60 is output to the setting unit 62.

The setting unit 62 has a function that, in a case in which it acquires body thickness information indicating a body thickness t of the subject W in the direction, in which the radiation R is transmitted, from the detector 14, derives the imaging conditions corresponding to the body thickness t indicated by the body thickness information and the imaging part indicated by the imaging part information and sets the imaging conditions in the radiation emitting device 12. For example, the setting unit 62 according to this embodiment sets, in the radiation emitting device, the imaging conditions for emitting the radiation R such that the dose of the radiation R transmitted through the imaging part is the same as a dose at a reference body thickness. The reference body thickness is the average value of body thicknesses determined for each imaging part. In addition, it is preferable that the reference body thickness is determined according to at least one of, for example, the race of the subject W, the age of the subject W, the gender of the subject W, or the ratio of muscle to fat in the subject W.

Specifically, the setting unit 62 acquires the body thickness information indicating the body thickness t of the subject W which has been input from the detector 14 through the I/F unit 54 by wireless communication. For example, in this embodiment, correspondence relationship information 53 indicating the correspondence relationship among the body thickness, the tube voltage value, and the mAs value illustrated in Fig. 5 is stored in the storage unit 52 in advance. The correspondence relationship information 53 illustrated in Fig. 5 is provided for each imaging part. The setting unit 62 derives the tube voltage value and the mAs value associated with the acquired body thickness information as the imaging conditions with reference to the correspondence relationship information corresponding to the imaging part indicated by the imaging part information input from the acquisition unit 60 and outputs the tube voltage value and the mAs value to the radiation emitting device 12.

In addition, the method by which the setting unit 62 derives the imaging conditions is not limited to the above-described method. For example, the imaging conditions predetermined according to the imaging part and the reference body thickness may be corrected according to the body thickness t acquired from the detector 14 to derive the imaging conditions to be set in the radiation emitting device 12. Further, for example, the imaging conditions predetermined according to the reference body thickness may be corrected according to the imaging part acquired by the acquisition unit 60 and the body thickness t acquired from the detector 14 to derive the imaging conditions to be set in the radiation emitting device 12. Furthermore, for example, the imaging conditions corresponding to the body thickness t acquired from the detector 14 may be corrected according to the imaging part acquired by the acquisition unit 60 to derive the imaging conditions to be set in the radiation emitting device 12.

In addition, in this embodiment, the aspect in which the tube voltage value and the mAs value are derived as the imaging conditions has been described. However, the imaging conditions derived by the setting unit 62 are not limited thereto. For example, instead of the mAs value, the irradiation time of the radiation R and a tube current value of the radiation source 20 may be derived as the imaging conditions.

Next, the operation of the console 10 according to this embodiment will be described with reference to the drawings.

In a case in which a radiographic image is captured, the subject W is positioned at a position facing the imaging surface 32A of the imaging table 32 as illustrated in Fig. 2. Then, the jaw 72A of the detector 14 is inserted between the subject W and the imaging table 32 in a state in which it is in contact with the subject W. In addition, the user slides the jaw 72B such that the subject W is interposed between the jaw 72A and the jaw 72B. The body thickness t of the subject W is displayed as the detection result on the display unit 41D. In a case in which the user operates the operation unit 41E in this state, the body thickness information indicating the body thickness t of the subject W is output as the detection result to the console 10. In this way, the user detects the body thickness t of the subject W.

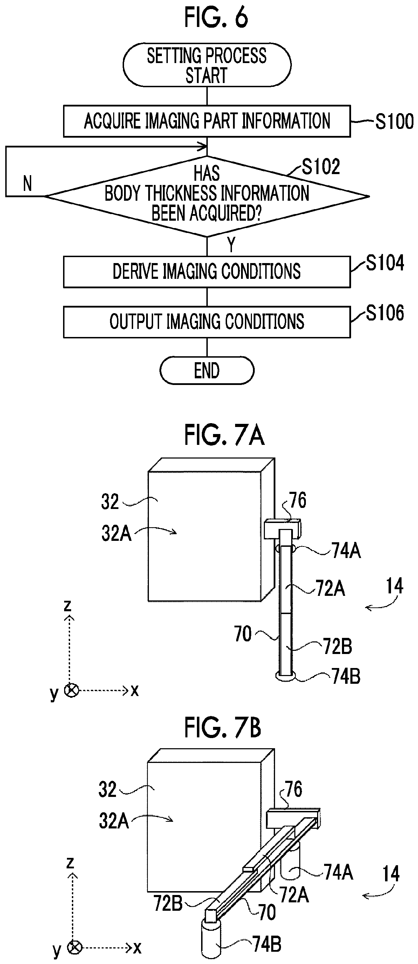

Meanwhile, in the console 10 according to this embodiment, the CPU 50A of the control unit 50 executes the setting program 51 stored in the ROM 50B to perform a setting process whose example is illustrated in Fig. 6. Fig. 6 is a flowchart illustrating an example of the flow of the setting process performed in the console 10 according to this embodiment. In addition, the timing when the CPU 50A performs the setting process is not limited, and the CPU 50A may perform the setting process at any timing. For example, the setting process may be performed at the timing when an instruction input from the user by the operation of the operation unit 56 after the positioning of the subject W ends is received or the timing when an instruction to emit the radiation R is received from the user.

In Step S100 of Fig. 6, the acquisition unit 60 acquires the imaging part information from the imaging menu as described above.

Then, in Step S102, the setting unit 62 determines whether or not the body thickness information has been acquired. The determination result in Step S102 is "No" until the body thickness information indicating the body thickness t of the subject W input from the detector 14 is acquired. On the other hand, in a case in which the body thickness information has been acquired, the determination result in Step S102 is "Yes", and the process proceeds to Step S104.

In Step S104, the setting unit 62 derives the imaging conditions as described above. Specifically, the setting unit 62 derives the tube voltage value and the mAs value corresponds to the imaging part indicated by the acquired imaging part information and the body thickness t indicated by the acquired body thickness information, with reference to the correspondence relationship information 53 stored in the storage unit 52.

Then, in Step S106, the setting unit 62 outputs the imaging conditions derived in Step S104 to the radiation emitting device 12 through the I/F unit 54. Then, in the radiation emitting device 12, the tube voltage value and the mAs value are set as the imaging conditions for emitting the radiation R. In a case in which the process in Step S106 ends, the setting process illustrated in Fig. 6 ends.

In addition, the aspect in which the detector 14 is a digital caliper that is provided separately from the imaging table 32 has been described. However, the form of the detector 14 is not limited to this aspect. For example, the detector 14 may have the forms described in the following Modification Examples 1 to 3 as long as it can come into contact with the subject W, detect the body thickness t, and output the body thickness information indicating the body thickness t as the detection result to the console 10.

(Modification Example 1)

The detector 14 may be integrated with the imaging table 32. Figs. 7A to 7D illustrate an example of the detector 14 as a foldable digital caliper which is provided integrally with the imaging table 32. In addition, for the sake of simplification of illustration, the display unit 41D and the operation unit 41E are not illustrated in Figs. 7A to 7D.

Fig. 7A illustrates a state in which the detector 14 is folded as an initial state. The detector 14 according to this modification example is attached to the imaging table 32 by a support portion 76. The support portion 76 is expanded and contracted in the x direction (lateral direction in Fig. 7A). The main scale 70 has a leading end attached to the support portion 76 and is parallel to the imaging surface 32A in the initial state. The jaw 72A and the jaw 72B are folded on the main scale 70. In addition, a grip 74A that is held by the user is provided at an end portion of the jaw 72A, and a grip 74B that is held by the user is provided at an end portion of the jaw 72B.

In a case in which the body thickness t of the subject W is detected, the user extends the support portion 76 according to the width of the subject W and separates the main scale 70 from the imaging table 32. Further, the user pulls up a side of the main scale 70 which is opposite to a side connected to the support portion 76 in the z-axis direction (upward direction in Fig. 7A) such that the detector 14 is in a state illustrated in Fig. 7B.

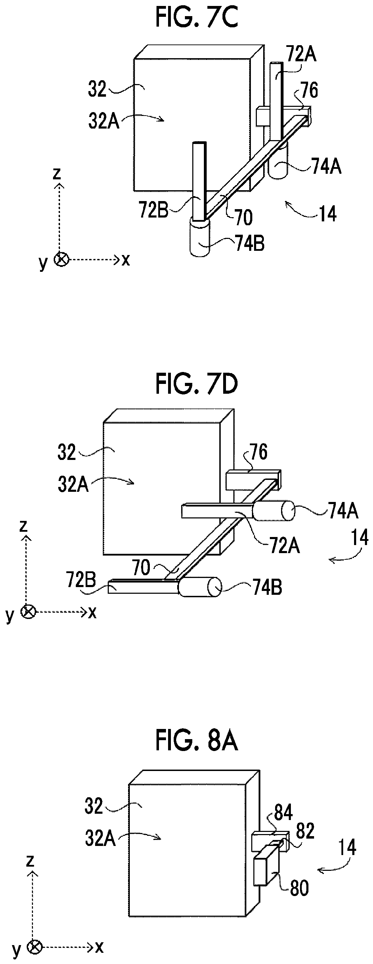

Furthermore, the user pulls up the leading end of the folded jaws 72A and 72B in the z-axis direction (upward direction in Fig. 7B) such that the detector 14 is in a state illustrated in Fig. 7C. In addition, the user tilts the leading ends of the jaws 72A and 72B toward the imaging table 32 such that the detector 14 is in a state illustrated in Fig. 7D. In a case in which the subject W is positioned on the imaging table 32, the user holds the grip 74B and moves the jaw 72B along the main scale 70 to bring the jaw 72B into contact with the surface of the subject W. In addition, the user holds the grip 74A and moves the jaw 72A along the main scale 70 to bring the jaw 72A into contact with the surface of the subject W. The interval between the jaw 72A and the jaw 72B is displayed on the display unit 41D according to the movement of the jaw 72A and the jaw 72B. In a case in which the user operates the operation unit 41E in this state, the interval between the jaw 72A and the jaw 72B is output as the body thickness information indicating the body thickness t of the subject W to the console 10.

In a case in which the detection of the body thickness t of the subject W ends in this way, the above-described procedure is reversely performed to fold the detector 14. In addition, the jaw 72A, the jaw 72B, and the like may be located at positions where they are not included in a radiographic image before the radiographic image is captured, specifically, before the radiation R is emitted from the radiation emitting device 12. The detector 14 may be returned to the initial state illustrated in Fig. 7A after the capture of the radiographic image is ended.

The interval between the jaw 72A and the jaw 72B is measured in this way, which makes it possible to more accurately measure the body thickness t of the subject W, for example, even in a case in which the imaging part is the lumbar vertebra or the like and the imaging part of the subject W is not in contact with the imaging surface 32A of the imaging table 32.

In addition, the detector 14 may not include the jaw 72A. For example, in a case in which a body surface of the subject W is in contact with the imaging surface 32A of the imaging table 32, the detector 14 may detect an interval between the imaging surface 32A and the jaw 72B. Further, for example, in a case in which the imaging part is the lumbar vertebra or the like and the imaging part of the subject W is not in contact with the imaging surface 32A of the imaging table 32, the jaw 72B is brought into contact with the body surface of the subject W which faces the radiation emitting device 12 to detect the interval between the imaging surface 32A of the imaging table 32 and the body surface of the subject W which faces the radiation emitting device 12. Furthermore, the jaw 72B is brought into contact with the body surface of the subject W which faces the imaging surface 32A to detect the interval between the imaging surface 32A of the imaging table 32 and the body surface of the subject W which faces the imaging surface 32A. The detector 14 may output the difference between the detection results of two consecutive detection operations as the body thickness information indicating the body thickness t of the subject W.

(Modification Example 2)

Figs. 8A to 8C illustrate an example of the detector 14 as a digital measure which is provided integrally with the imaging table 32. Fig. 8A illustrates an initial state of the detector 14. The detector 14 according to this modification example is attached to the imaging table 32 by a support portion 84. The support portion 84 is expanded and contracted in the x direction (lateral direction in Fig. 8A). A leading end of a tape 82 that is accommodated in a main body 80 is attached to the support portion 84. The display unit 41D and the operation unit 41E (not illustrated) are provided on a side surface of the main body 80.

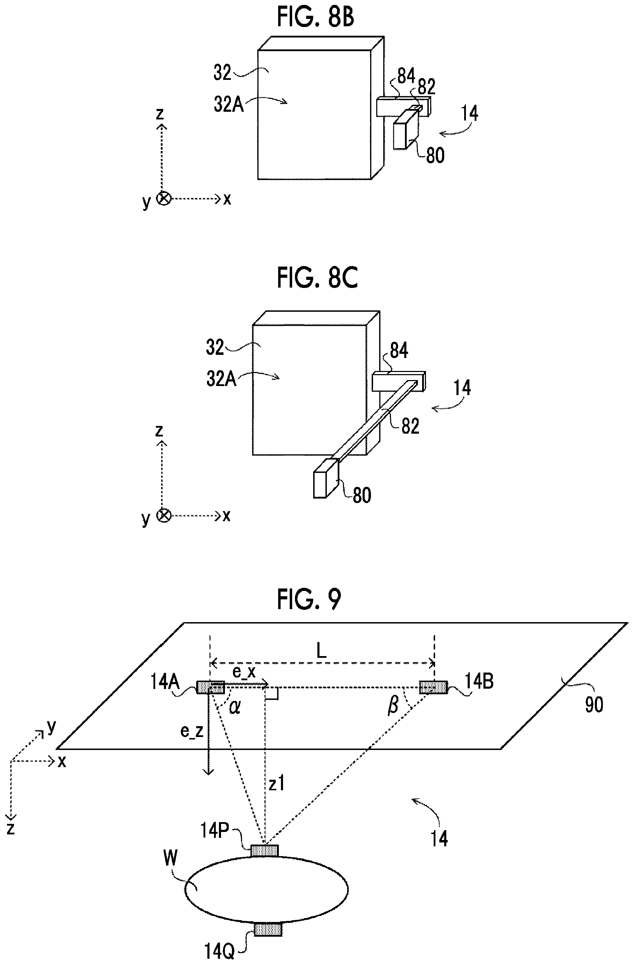

In a case in which the body thickness t of the subject W is detected, the user extends the support portion 84 according to the width of the subject W and separates the main body 80 from the imaging table 32 such that the detector 14 is in a state illustrated in Fig. 8B. In a case in which the user positions the subject W on the imaging table 32, as illustrated in Fig. 8C, the user moves the main body 80 in a direction toward the radiation emitting device 12 (a direction toward the front in Fig. 8C) along the y-axis direction to stretch the tape 82 such that an end portion of the tape 82 which is close to the main body 80 is at the same position as the body surface of the subject W. In the example illustrated in Figs. 8A to 8C, the length of the stretched tape 82 corresponds to the length from the imaging surface 32A of the imaging table 32 to the main body 80 of the detector 14. The length of the stretched tape 82 is displayed on the display unit 41D. In a case in which the user operates the operation unit 41E in this state, the length of the stretched tape 82 is output as the body thickness information indicating the body thickness t of the subject W to the console 10.

In a case in which the detection of the body thickness t of the subject Wends in this way, the above-described procedure is reversely performed to return the detector 14 to the initial state. In addition, the main body 80 and the like may be located at a position where they are not included in a radiographic image before the radiographic image is captured, specifically, before the radiation R is emitted from the radiation emitting device 12. The detector 14 may be returned to the initial state illustrated in Fig. 8A after the capture of the radiographic image is ended.

Further, the example in which the length of the stretched tape 82 corresponds to the length from the imaging surface 32A of the imaging table 32 to the main body 80 of the detector 14 has been described. However, the present disclosure is limited to this example. For example, as described in Modification Example 1, the detector 14 may output the difference between the detection results of two consecutive detection operations as the body thickness information indicating the body thickness t of the subject W.

(Modification Example 3)

Fig. 9 illustrates an example of a detector 14 having another form. The detector 14 illustrated in Fig. 9 comprises reference measurement devices 14A and 14B and a pair of measurement devices 14P and 14Q. The reference measurement devices 14A and 14B and the measurement devices 14P and 14Q can wirelessly communicate with each other. The reference measurement devices 14A and 14B are provided at known positions that serve as reference detection positions and are provided, for example, on a ceiling 90, a wall, or the like of a room in which the imaging table 32 is disposed. In addition, the pair of the measurement devices 14P and 14Q is disposed by the user in a state in which it is in contact with both sides of a portion in which the body thickness t of the subject W is measured, that is, a portion corresponding to the imaging part on the body surface. For example, the measurement devices 14P and 14Q are wearable measurement devices and are worn on both hands of the user or the fingers of both hands, respectively. The operation unit 41E is provided in each of the measurement devices 14P and 14Q.

In the measurement of the body thickness t of the subject W, in a case in which the user positions the subject W on the imaging table 32 (not illustrated in Fig. 9), the measurement devices 14P and 14Q are disposed at both ends of the imaging part in which the body thickness t of the subject W is measured, respectively, as illustrated in Fig. 9. For example, the user holds the imaging part of the subject W between hands having the measurement devices 14P and 14Q worn thereon to dispose each of the measurement devices 14P and 14Q at a measurement position. In a case in which the user operates either the operation unit 41E provided in the measurement device 14P or the operation unit 41E provided in the measurement device 14Q in this state, the distance between the measurement device 14P and the measurement device 14Q is output as the body thickness information indicating the body thickness t of the subject W to the console 10.

A method for detecting the distance between the measurement device 14P and the measurement device 14Q is not limited. For example, a triangulation method can be applied. An example of the detection method in this case will be described. In the example illustrated in Fig. 9, a distance L between the reference measurement device 14A and the reference measurement device 14B is known. Further, angles $\alpha$ and $\beta$ can be known by measurement. Therefore, a vector AP defined by the reference measurement device 14A and the measurement device 14P can be derived to detect the position of the measurement device 14P. Specifically, the measurement device 14P derives the vector AP as the position of the measurement device 14P from the following Expressions (1) to (4). In addition, in the following Expression (1), "AB" means a distance between the reference measurement device 14A and the reference measurement device 14B. In the following Expression (3), "AP" means a distance between the reference measurement device 14A and the measurement device 14P. Further, a unit vector $e\_x$ is a unit vector in the direction of the reference measurement devices 14A and 14B. Further, a unit vector $e\_z$ is a unit vector that is along a plane passing through the reference measurement devices 14A and 14B and the measurement device 14P and that is perpendicular to the unit vector $e\_x$. In addition, z1 is the length of a perpendicular line extending from the measurement device 14P to a straight line passing through the reference measurement devices 14A and 14B.

$$AB = L = z1/\tan\alpha + z1/\tan\beta \quad \cdots(1)$$

$$z1 = L \times \tan\alpha \times \tan\beta/(\tan\alpha + \tan\beta) \quad \cdots(2)$$

$$AP = z1/\sin\alpha = L \times \sin\beta/\sin(\alpha + \beta) \quad \cdots(3)$$

$$\text{Vector } AP = L \times \sin\beta/\sin(\alpha + \beta) \times (\cos\alpha \times e\_x + \sin\alpha \times e\_z) \quad \cdots(4)$$

In addition, similarly to the measurement device 14P, the measurement device 14Q can derive a vector AQ to detect the position of the measurement device 14Q. Any one of the measurement device 14P or the measurement device 14Q derives the difference between its own position and the position of the other measurement device from the vector AQ and the vector AP to detect the distance between the measurement device 14P and the measurement device 14Q. In addition, even in a case in which the measurement device 14Q is located on a plane different from the plane formed by the reference measurement devices 14A and 14B and the measurement device 14P, a three-dimensional vector can be defined by defining a unit vector e_z' and defining the relationship between the unit vector e_z' and the unit vector e_z. Therefore, even in a case in which the measurement device 14Q is located on a plane different from the plane formed by the reference measurement devices 14A and 14B and the measurement device 14P, it is possible to derive the position of the measurement device 14Q.

According to the detector 14 of this modification example, it is possible to reduce the size and weight of the measurement devices 14P and 14Q. Therefore, the user can easily handle the measurement devices 14P and 14Q. In addition, since the measurement devices 14P and 14Q are wearable devices, for example, the user can come into contact with each of both ends of the imaging part of the subject W, in which the body thickness t is measured, to measure the body thickness t. Therefore, according to the detector 14 of this modification example, it is possible to easily measure the body thickness t, regardless of, for example, a complicated shape or a location where the subject W is positioned.

In addition, in this modification example, the aspect in which two reference measurement devices 14A and 14B are provided as the reference measurement devices has been described. However, the number of reference measurement devices may be two or more and is not limited to two.

As described above, the console 10 according to each of the above-described embodiments comprises the CPU 50A as at least one processor. The CPU 50A acquires the imaging part information indicating the imaging part of the subject W whose radiographic image is captured by the radiation R emitted from the radiation emitting device 12. Further, in a case in which the CPU 50A acquires the body thickness information indicating the body thickness t of the subject W in the direction in which the radiation R is transmitted from the detector 14 that comes into contact with the subject W and detects the body thickness t, the CPU 50A derives the imaging conditions corresponding to the body thickness t indicated by the body thickness information and the imaging part indicated by the imaging part information and sets the imaging conditions in the radiation emitting device 12.

The setting unit 62 of the console 10 according to this embodiment can set, in the radiation emitting device, the imaging conditions for emitting the radiation R such that the dose of the radiation transmitted through the imaging part is the same as the dose at the reference body thickness, on the basis of the body thickness and the imaging part of the subject W.

Therefore, according to the console 10 of this embodiment, it is possible to easily set the imaging conditions corresponding to the imaging part and the body thickness of the subject W. In addition, according to the console 10 of this embodiment, appropriate tube voltage kV and mAs value corresponding to the imaging part and the body thickness t of the subject W are automatically set. Therefore, it is possible to reduce the burden on the user for setting.

In addition, in this embodiment, the detector that comes into contact with the subject W and detects the body thickness t is used as the detector that detects the body thickness t of the subject W. Therefore, the body thickness t can be detected with higher accuracy than that in a case in which a detector that detects the body thickness t in a non-contact manner is used. Specifically, since the detector comes into contact with the portion in which the body thickness t of the subject W is measured, it is possible to measure the body thickness t regardless of, for example, the color, shape, thickness, and material of the clothes of the subject W. Therefore, it is possible to improve the reproducibility of detection and to improve robustness to a variation in the clothes of the subject W.

In addition, in each of the above-described embodiments, the aspect in which the console 10, the radiation emitting device 12, and the radiography apparatus 16 are stationary in the radiography system 1 has been described. However, the system of the radiography system 1 is not limited to this aspect. For example, a mobile cart, that is, a nursing cart may be used as the radiography system 1.

Further, in each of the above-described embodiments, the aspect in which the console 10 is an example of the setting device according to the present disclosure has been described. However, devices other than the console 10 may have the functions of the setting device according to the present disclosure. In other words, for example, the radiation emitting device 12, the radiography apparatus 16, or an external device other than the console 10 may have some or all of the functions of the acquisition unit 60 and the setting unit 62.

In addition, in each of the above-described embodiments, for example, the following various processors can be used as a hardware structure of processing units performing various processes such as the acquisition unit 60 and the setting unit 62. The various processors include, for example, a programmable logic device (PLD), such as a field programmable gate array (FPGA), that is a processor whose circuit configuration can be changed after manufacture and a dedicated electric circuit, such as an application specific integrated circuit (ASIC), that is a processor having a dedicated circuit configuration designed to perform a specific process, in addition to the CPU that is a general-purpose processor which executes software (programs) to function as various processing units as described above.

One processing unit may be configured by one of the various processors or a combination of two or more processors of the same type or different types (for example, a combination of a plurality of FPGAs or a combination of a CPU and an FPGA). In addition, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As such, various processing units are configured by using one or more of the various processors as a hardware structure.

In addition, specifically, an electric circuit (circuitry) obtained by combining circuit elements, such as semiconductor elements, can be used as the hardware structure of the various processors.

Further, in each of the above-described embodiments, the aspect in which the setting program 51 is stored (installed) in the storage unit 52 in advance has been described. However, the present disclosure is not limited thereto. The setting program 51 may be recorded on a recording medium, such as a compact disc read only memory (CD-ROM), a digital versatile disc read only memory (DVD-ROM), or a universal serial bus (USB) memory, and then provided. In addition, the setting program 51 may be downloaded from an external device through the network.

The disclosure of JP2020-161415 filed on September 25, 2020 is incorporated herein by reference in its entirety.

All of the documents, the patent applications, and the technical standards described in the specification are incorporated by reference herein to the same extent as it is specifically and individually stated that individual documents, patent applications, and technical standards are incorporated by reference.

Explanation of References

1: radiography system
10: console
12: radiation emitting device
14: detector
14A, 14B: reference measurement device
14P, 14Q: measurement device
16: radiography apparatus
20: radiation source
21A, 31A: control unit
21B, 31B: storage unit
21C, 31C, 41C: I/F unit
21D, 41D: display unit
41E: operation unit
22: focus
24: collimator
28: imaging element

30: radiation detector
30A: detection surface
32: imaging table
32A: imaging surface
36: base
50: control unit
50A: CPU
50B: ROM
50C: RAM
51: setting program
52: storage unit
54: I/F unit
56: operation unit
58: display unit
59: bus
60: acquisition unit
62: setting unit
70: main scale
72A, 72B: jaw
74A, 74B: grip
76, 84: support portion
80: main body
82: stretched tape
90: ceiling
e_x, e_z: unit vector
L: distance
R: radiation
t: body thickness
W: subject
z1: perpendicular line
α, β: angle

## Claims

1. A setting device comprising: at least one processor, wherein the processor is configured to:

   acquire imaging part information indicating an imaging part of a subject whose radiographic image is captured by radiation emitted from a radiation emitting device; and
   derive, in a case in which body thickness information indicating a body thickness of the subject in a direction in which the radiation is transmitted is acquired from a detector that comes into contact with the subject and detects the body thickness, imaging conditions corresponding to the body thickness indicated by the body thickness information and the imaging part indicated by the imaging part information and set the imaging conditions in the radiation emitting device.

2. The setting device according to claim 1, wherein the detector is integrated with an imaging table for capturing the radiographic image.

3. The setting device according to claim 1 or 2, wherein the processor is configured to wirelessly acquire the body thickness information from the detector.

4. The setting device according to any one of claims 1 to 3, wherein the detector is a digital caliper.

5. The setting device according to any one of claims 1 to 3, wherein the detector is a digital measure.

6. The setting device according to any one of claims 1 to 5, wherein:

   the detector includes a reference measurement device that is provided at a reference position and a pair of measurement devices that comes into contact with both sides of a portion in which the body thickness of the subject is measured, and

the processor is configured to acquire positional information indicating a position of each of the pair of measurement devices as the body thickness information and derives the body thickness on the basis of the body thickness information and the reference position.

7. The setting device according to any one of claims 1 to 6, wherein the imaging conditions are at least one of a tube voltage value, a tube current value, an irradiation time, or a mAs value.

8. The setting device according to any one of claims 1 to 7, wherein the processor is configured to acquire the imaging part information from an imaging menu.

9. A setting method executed by a processor, the setting method comprising:

acquiring imaging part information indicating an imaging part of a subject whose radiographic image is captured by radiation emitted from a radiation emitting device; and

deriving, in a case in which body thickness information indicating a body thickness of the subject in a direction in which the radiation is transmitted is acquired from a detector that comes into contact with the subject and detects the body thickness, imaging conditions corresponding to the body thickness indicated by the body thickness information and the imaging part indicated by the imaging part information and setting the imaging conditions in the radiation emitting device.

10. A setting program that causes a processor to execute a process comprising:

acquiring imaging part information indicating an imaging part of a subject whose radiographic image is captured by radiation emitted from a radiation emitting device; and

deriving, in a case in which body thickness information indicating a body thickness of the subject in a direction in which the radiation is transmitted is acquired from a detector that comes into contact with the subject and detects the body thickness, imaging conditions corresponding to the body thickness indicated by the body thickness information and the imaging part indicated by the imaging part information and setting the imaging conditions in the radiation emitting device.

## FIG. 1

## FIG. 2

# FIG. 3

# FIG. 4

# FIG. 5

| BODY THICKNESS (cm) | TUBE VOLTAGE VALUE (kV) | mAs VALUE | |
|---|---|---|---|
| 20 | 80 | 30 | 53 |
| 25 | 90 | 28 | |
| ... | ... | ... | |

## FIG. 6

```
┌─────────────────────┐
│   SETTING PROCESS    │
│       START          │
└─────────────────────┘
          │
┌─────────────────────────────────┐
│  ACQUIRE IMAGING PART INFORMATION │──S100
└─────────────────────────────────┘
          │
          ▼
      ╱─────────╲
  N  ╱    HAS     ╲  S102
◄───╱ BODY THICKNESS INFORMATION ╲
    ╲  BEEN ACQUIRED?  ╱
      ╲─────────╱
          │ Y
┌─────────────────────────────────┐
│    DERIVE IMAGING CONDITIONS     │──S104
└─────────────────────────────────┘
          │
┌─────────────────────────────────┐
│    OUTPUT IMAGING CONDITIONS     │──S106
└─────────────────────────────────┘
          │
      ┌───────┐
      │  END  │
      └───────┘
```

## FIG. 7A

## FIG. 7B

## FIG. 7C

## FIG. 7D

## FIG. 8A

## FIG. 8B

## FIG. 8C

## FIG. 9

## INTERNATIONAL SEARCH REPORT

| International application No. |
| --- |
| **PCT/JP2021/028493** |

| A. | CLASSIFICATION OF SUBJECT MATTER |
| --- | --- |

***A61B 6/00***(2006.01)i
FI:   A61B6/00 320M; A61B6/00 390B

According to International Patent Classification (IPC) or to both national classification and IPC

| B. | FIELDS SEARCHED |
| --- | --- |

Minimum documentation searched (classification system followed by classification symbols)

A61B6/00-6/14

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 6-277204 A (HITACHI MEDICAL CORP) 04 October 1994 (1994-10-04) <br> paragraphs [0005], [0008]-[0010] | 1-10 |
| Y | JP 5-15521 A (KONICA CORP) 26 January 1993 (1993-01-26) <br> paragraphs [0011]-[0017] | 1-5, 7-10 |
| Y | JP 2009-207527 A (FUJIFILM CORP) 17 September 2009 (2009-09-17) <br> paragraphs [0019]-[0020], [0023] | 1-10 |
| Y | US 2020/0289207 A1 (MICRO C, LLC) 17 September 2020 (2020-09-17) <br> paragraphs [0247]-[0254] | 6 |
| A | JP 3-224545 A (SHIMADZU CORP) 03 October 1991 (1991-10-03) <br> entire text, all drawings | 1-10 |
| A | JP 2015-43959 A (FUJIFILM CORP) 12 March 2015 (2015-03-12) <br> entire text, all drawings | 1-10 |
| A | JP 4-241842 A (KONICA CORP) 28 August 1992 (1992-08-28) <br> entire text, all drawings | 1-10 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **16 September 2021** | **05 October 2021** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
|---|
| **PCT/JP2021/028493** |

### C. DOCUMENTS CONSIDERED TO BE RELEVANT

| Category* | Citation of document, with indication, where appropriate, of the relevant passages | Relevant to claim No. |
|---|---|---|
| A | JP 2009-261762 A (HITACHI MEDICAL CORP) 12 November 2009 (2009-11-12)<br>entire text, all drawings | 1-10 |
| A | JP 5-253219 A (HITACHI MEDICAL CORP) 05 October 1993 (1993-10-05)<br>entire text, all drawings | 1-10 |
| A | US 2018/0206810 A1 (SAMSUNG ELECTRONICS CO., LTD.) 26 July 2018 (2018-07-26)<br>entire text, all drawings | 1-10 |

Form PCT/ISA/210 (second sheet) (January 2015)

**INTERNATIONAL SEARCH REPORT**
**Information on patent family members**

International application No.

**PCT/JP2021/028493**

| Patent document cited in search report | | | Publication date (day/month/year) | Patent family member(s) | | | Publication date (day/month/year) |
|---|---|---|---|---|---|---|---|
| JP | 6-277204 | A | 04 October 1994 | (Family: none) | | | |
| JP | 5-15521 | A | 26 January 1993 | (Family: none) | | | |
| JP | 2009-207527 | A | 17 September 2009 | (Family: none) | | | |
| US | 2020/0289207 | A1 | 17 September 2020 | (Family: none) | | | |
| JP | 3-224545 | A | 03 October 1991 | (Family: none) | | | |
| JP | 2015-43959 | A | 12 March 2015 | US entire text, all drawings | 2016/0140720 | A1 | |
| JP | 4-241842 | A | 28 August 1992 | (Family: none) | | | |
| JP | 2009-261762 | A | 12 November 2009 | (Family: none) | | | |
| JP | 5-253219 | A | 05 October 1993 | (Family: none) | | | |
| US | 2018/0206810 | A1 | 26 July 2018 | EP KR 10-2018-0086709 | 3351176 | A1 A | |

Form PCT/ISA/210 (patent family annex) (January 2015)

**REFERENCES CITED IN THE DESCRIPTION**

**Patent documents cited in the description**

- JP 2006218142 A

- JP 2020161415 A